# EUROPÄISCHE PATENTSCHRIFT

(11) **EP 3 590 917 B1**
(45) Veröffentlichungstag und Bekanntmachung des Hinweises auf die Patenterteilung: **21.04.2021**
(21) Anmeldenummer: 18181805.5
(22) Anmeldetag: 05.07.2018
(51) Int. Cl.: C07C 68/02, C07C 69/96

(54) **VERFAHREN ZUR HERSTELLUNG VON DIALKYLDICARBONATEN MITTELS AMINOXIDE**
METHOD FOR THE PREPARATION OF DIALKYLDICARBONATES BY AMINE OXIDES
PROCÉDÉ DE PRODUCTION DE BICARBONATES DE DIALKYLE À L'AIDE DE L'OXYDE D'AMINE

(43) Veröffentlichungstag der Anmeldung: 08.01.2020
(73) Patentinhaber: LANXESS Deutschland GmbH, 50569 Köln (DE)
(72) Erfinder: HOFMANN, Christoph, 51061 Köln (DE)

(56) Entgegenhaltungen:
- WO-A1-2005/110964
- DE-A1- 1 418 849
- DE-B- 1 210 853

## Beschreibung

Die vorliegende Erfindung betrifft ein Verfahren zur Herstellung von Dialkyldicarbonaten aus den entsprechenden Chlorameisensäurealkylestern unter Verwendung spezieller Aminoxide als Katalysatoren.

Dialkyldicarbonate finden z.B. als Katalysatoren zur Oxidation von sterisch anspruchsvollen Aminen, als Bestandteile von Elektrolytflüssigkeiten oder als Bestandteile von antimikrobiellen Reagenzien Verwendung. Dialkyldicarbonate werden in der Literatur auch als Dialkylpyrocarbonate bezeichnet.

Aus DE-B 1210 853 ist bekannt, Kohlensäure- oder Carbonsäurehalogenide mit organischen Hydroxylverbindungen oder deren Alkali- oder Erdalkalisalzen und organischen, mit Wasser nicht mischbaren Lösungsmitteln und mindestens äquivalenten Mengen an Alkali- oder Erdalkalihydroxiden oder -carbonaten in einem zweiphasigen System, sowie unter Verwendung katalytischer Mengen an tertiären Aminen oder ihren Quaternierungsprodukten, umzusetzen, wobei als Amine oder deren Quaternierungsprodukte solche eingesetzt werden, die mindestens eine an Stickstoff gebundene ω-Hydroxyalkyl-, ω-Hydroxyalkyläther- oder ω-Hydroxyalkylpolyäthergruppe tragen.

In DE-A 1 418 849 werden tertiäre Amine als besonders geeignete Katalysatoren für die Herstellung von Säurederivaten beschrieben, deren tertiäre Stickstoffatome sterisch nicht gehindert sind, ausgenommen tertiäre Amine, die als Substituenten am Stickstoff ebensolche ω-Hydroxyalkyl-, ω-Hydroxyalkyläther- oder ω-Hydroxyalkylpolyäthergruppe tragen. Neben Triethylamin und Tri-n-butylamin kommen hier deshalb Amine zum Einsatz, die mindestens eine Methylgruppe am Stickstoff tragen, wie z.B. N-Methyl-di-n-stearylamin. Diese Katalysatoren besitzen aber u.a. den Nachteil, dass sie nicht nur die Bildung, sondern auch die Zersetzung des Produktes katalysieren, was zu einer Verringerung der Ausbeute führt. Zudem sind einige dieser Katalysatoren toxisch, werden im Abwasser schlecht abgebaut und lassen sich vom Reaktionsgemisch aufgrund eigener Zersetzungen während der Reaktion schlecht abtrennen.

Aus der EP-A 1747185 ist ein Verfahren zur Herstellung von Dialkyldicarbonaten aus Halogenameisensäurealkylestern durch Umsetzung mit Alkali- oder Erdalkalihydroxiden oder -carbonaten bekannt, bei dem langkettige tertiäre C₆-C₂₅-Alkylamine eingesetzt werden. Auch mit diesen Katalysatoren war die Ausbeute an Produkt noch nicht vollständig zufriedenstellend.

Es bestand daher weiterhin Bedarf an einem Herstellungsverfahren, welches das Zielprodukt in hoher Ausbeute liefert.

Überraschenderweise wurde gefunden, dass Dialkyldicarbonate aus Halogenameisensäurealkylestern durch Umsetzung mit Alkali- oder Erdalkalihydroxiden oder -carbonaten besonders vorteilhaft erhalten werden können, wenn man als Katalysator spezielle Aminoxide der Formel (I) einsetzt. Diese zeichnen sich durch eine hohe katalytische Aktivität aus, ohne zersetzend auf das Endprodukt zu wirken. Zudem ist die Ausbeute an Dialkyldicarbonate mit den Verbindungen der Formel (I) höher als mit herkömmlichen tertiären Aminen.

Gegenstand der vorliegenden Erfindung ist demnach ein Verfahren zur Herstellung von Dialkyldicarbonaten durch Umsetzung der entsprechenden Halogenameisensäurealkylester mit Alkali- oder Erdalkalihydroxiden und/oder -carbonaten in Gegenwart von mit Wasser nicht mischbaren organischen Lösungsmitteln und in Gegenwart eines Katalysators dadurch gekennzeichnet ist, dass als Katalysator mindestens eine Verbindung der Formel (I) 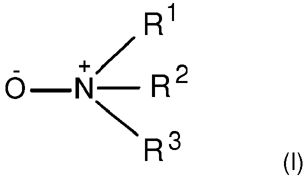 worin
R¹ und R²= geradkettiges oder verzweigtes C₁-C₆-Alkyl,
R³ = geradkettiges oder verzweigtes C₁₀-C₂₂-Alkyl,
wobei R¹ und R² jeweils unabhängig voneinander sind und gleich oder verschieden sein können, eingesetzt wird.

Bevorzugt werden bei der Durchführung des erfindungsgemäßen Verfahrens als Katalysator eine Verbindung der Formel (I) eingesetzt, worin R¹ und R² = Methyl, Ethyl, n-Propyl, s-Propyl, n-Butyl, s-Butyl, i-Butyl oder t-Butyl ist und R³ = geradkettiges oder verzweigtes C₁₀-C₁₈-Alkyl.

Besonders bevorzugt werden als Katalysatoren Verbindungen der Formel (I) mit R¹ und R²= Methyl und R³ = C₁₂-C₁₈-Alkyl eingesetzt.

In einer Ausführungsform des erfindungsgemäßen Verfahrens wird als Katalysator eine Verbindung der Formel (I) eingesetzt, bei dem R³ jeweils geradkettiges oder verzweigtes Dodecanyl, Undecanyl, Tridecanyl, Tetradecanyl, Pentadecanyl, Hexadecanyl, Heptadecanyl, Octadecanyl, Nonadecanyl, Eicosanyl, Icosanyl, Heneicosanyl oder Dodoconyl bedeutet. Bevorzugt ist R³ = Dodecanyl, Tetradecanyl, Hexadecanyl und Octadecanyl.

In einer weiteren Ausführungsform des erfindungsgemäßen Verfahrens wird als Katalysator eine Verbindung der Formel (I) bei der R¹ und R² = Methyl und R³ = Dodecanyl, Tetradecanyl, und Hexadecanyl ist.

Bei der Durchführung des erfindungsgemäßen Verfahrens können auch beliebige Mischungen der Katalysatoren eingesetzt werden.

In einer weiteren bevorzugten Ausführungsform werden als Katalysatoren Mischungen von Verbindungen der Formel (I) eingesetzt, die verschiedene Reste R³ mit geradkettigem oder verzweigtem C₁₀-, C₁₁-, C₁₂-, C₁₃-, C₁₄- C₁₅- C₁₆-, C₁₇- oder C₁₈-Alkyl aufweisen.

Noch weiter bevorzugt werden Mischungen von Verbindungen der Formel (I) eingesetzt, die verschiedene Reste R³ mit geradkettigem oder verzweigtem C₁₂-, C₁₄- und C₁₆-Alkyl beinhalten und bei denen R¹ und R² = Methyl ist.

In einer weiteren bevorzugten Ausführungsform werden als Katalysatoren Mischungen der Verbindungen der Formel (I) eingesetzt, die verschieden Reste R³ mit geradkettigem oder verzweigtem C₁₂-, C₁₄- und C₁₆-Alkyl und R¹ und R² = Methyl aufweisen und bei denen der Gehalt der Verbindung mit R³ = C₁₂-Alkyl von 60 Gew.% und 80 Gew.% und der Gehalt der Verbindung mit R³ = C₁₄-Alkyl von 19 Gew. % und 30 Gew. % und der Gehalt der Verbindung mit R³ = C₁₆-Alkyl von 1 Gew. % und 10 Gew.% bezogen auf das Gesamtgewicht der eingesetzten Verbindung der Formel (I) ist.

In einer weiteren bevorzugten Ausführungsform werden als Verbindungen der Formel (I) Mischungen von Lauryldimethyldiaminoxid (*N,N*-Dimethyldodecan-1-aminoxid), Myristaminoxid (*N,N-*Dimethyltetradecan-1-aminoxid) und Hexadecanyldimethylaminoxid (*N,N*-Dimethylhexadecan-1-aminoxid) eingesetzt. Bevorzugt werden diese Katalysatoren in einem Gehalt von 60 Gew. % bis 80 Gew.% Lauryldimethylaminoxid, 19 Gew. % bis 30 Gew. % Myristaminoxid und 1 Gew. % bis 10 Gew. % Hexadecanyldimethylaminoxid, bezogen auf das Gesamtgewicht der Verbindungen der Formel (I), eingesetzt.

In einer weiteren bevorzugten Ausführungsform können als Katalysatoren Mischungen der Verbindungen der Formel (I) eingesetzt werden, bei denen die Reste R¹, R² und R³ im Rahmen der obigen Offenbarung in beliebiger Art kombiniert werden. In einer weiteren bevorzugten Ausführungsform werden die Katalysatoren in Wasser, gegebenenfalls in Gegenwart eines mit Wasser mischbaren Lösungsmittel, vor der Verwendung in dem erfindungsgemäßen Verfahren gelöst. Als mit Wasser mischbaren Lösungsmitteln werden bevorzugt Alkohole, wie insbesondere Glykole, verwendet. Bevorzugt liegen die Verbindungen der Formel (I) in einer Konzentration zwischen 25 Gew.% bis 75 Gew.% im Wasser oder in den Mischungen mit Wasser und dem mit Wasser mischbaren Lösungsmittel, vor.

Die zur Herstellung der Katalysatoren üblicherweise eingesetzten tertiären Amine werden leicht durch Wasserstoffperoxid oder eine Peroxycarbonsäure zu Aminoxiden oxidiert. Katalysatoren wie z.B. *N,N*-Dimethyldodecan-1-aminoxid sind kommerziell erhältlich und werden z.B. unter dem Namen Barlox 12 oder Barlox 1260 von der Fa. Lonza vertrieben Ihre Herstellungsverfahren sind ferner dem Fachmann bekannt.

Vorzugsweise dient das erfindungsgemäße Verfahren zur Herstellung von Dialkyldicarbonaten der Formel (II) 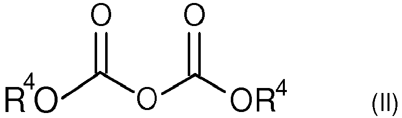 worin
- R⁴: für geradkettiges oder verzweigtes C₁-C₂₀-Alkyl steht,
durch Umsetzung von Halogenameisensäurealkylester der Formel (III) 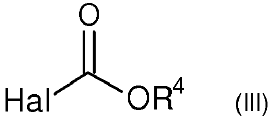 worin
- Hal: für Halogen, bevorzugt für F, Cl, Br, I, insbesondere für Chlor steht, und
- R⁴: für geradkettiges oder verzweigtes C₁-C₂₀-Alkyl steht.

In den Formeln (II) und (III) steht R⁴ vorzugsweise für geradkettiges oder verzweigtes C₁-C₈-Alkyl, besonders bevorzugt für einen Rest -CH-R⁵R⁶, wobei R⁵ und R⁶ unabhängig voneinander für H oder für geradkettiges oder verzweigtes C₁-C₇-Alkyl stehen. Insbesondere steht R⁴ für Methyl, Ethyl, n-Propyl, iso-Propyl, n-Butyl oder i-Butyl. Insbesondere bevorzugt steht R⁴ für Methyl, so dass Dimethyldicarbonat als Verbindung der Formel (II) erhalten wird.

Als Alkali- oder Erdalkalihydroxide oder -carbonate kommen beispielsweise LiOH, NaOH, KOH, LiCO₃, Na₂CO₃, K₂CO₃ in Frage. Vorzugsweise werden Alkalihydroxide verwendet, wie Natrium- und Kaliumhydroxid, die vorzugsweise in Form von wässrigen Lösungen zum Einsatz gelangen. Beispielsweise kann man 1 bis 50 gew.-%ige wässrige Alkalihydroxidlösungen verwenden. Bevorzugt sind 5 bis 35 gew.-%ige Lösungen, besonders bevorzugt 10 bis 25 gew.%ige Lösungen. Die Alkali- oder Erdalkalihydroxide oder -carbonate werden vorzugsweise in Mengen von 80 bis 120 Mol-% bezogen auf eingesetzten Halogenameisensäureester eingesetzt. Besonders bevorzugt liegt diese Menge im Bereich von 90 bis 115 Mol-%, ganz besonders bevorzugt im Bereich von 95 bis 115 Mol-%.

Als mit Wasser nicht mischbare organische Lösungsmittel kommen beispielsweise aliphatische und aromatische Kohlenwasserstoffe, halogenierte Kohlenwasserstoffe, mit Wasser nicht mischbare Ether oder Ester sowie Dialkylcarbonate in Frage. Bevorzugt sind Cyclohexan, Toluol, Xylol, Methylenchlorid und Diethylether, insbesondere Toluol und Methylenchlorid. Ganz besonders bevorzugt wird Toluol eingesetzt.

Das mit Wasser nicht mischbare organische Lösungsmittel kann man beispielsweise in Mengen von 20 bis 90 Gew.-%, bevorzugt von 30 bis 80 Gew.-%, besonders bevorzugt von 40 bis 80 Gew.-% bezogen auf den Halogenameisensäureester der Formel (III) einsetzen.

Der Katalysator der Formel (I) wird im Allgemeinen bezogen auf Halogenameisensäureester der Formel (III), in einer Menge von 0,1 bis 7 Mol-%, vorzugsweise von 0,5 bis 5 Mol-% eingesetzt. Noch weiter bevorzugt wird der Katalysator der Formel (I) in einer Menge von 0,5 bis 3 Mol-% eingesetzt.

Das erfindungsgemäße Verfahren kann in einem Druckbereich von 1 bis 10 bar, vorzugsweise von 1 bis 1,5 bar durchgeführt werden.

Die Reaktionstemperatur kann beispielsweise zwischen -10°C und der Siedetemperatur (bei Normaldruck) des eingesetzten Halogenameisensäureesters liegen. Vorzugsweise liegt sie im Bereich 0 bis 50°C. Noch weiter bevorzugt liegt die Reaktionstemperatur zwischen 15°C und 19°C.

Es ist vorteilhaft, während der Durchführung des erfindungsgemäßen Verfahrens für eine gute Durchmischung zu sorgen, z.B. durch Einsatz von Rührwerken, Strömungsstörern oder Umwälzpumpen.

Das erfindungsgemäße Verfahren kann sowohl batchweise als auch kontinuierlich durchgeführt werden. Bei batchweiser Arbeitsweise wird die Umsetzung bevorzugt in einem Rührkessel durchgeführt. Die Reaktion ist hierbei je nach Größe des Ansatzes und der vorhandenen Kühlleistung im allgemeinen nach 10 Minuten bis 3 Stunden beendet.

Vorzugsweise wird das erfindungsgemäße Verfahren kontinuierlich unter Verwendung eines Rührkessels, einer Rührkesselkaskade oder eines Rohrreaktors durchgeführt. Hierbei beträgt die mittlere Verweilzeit im Reaktor in der Regel zwischen 1 und 60 Minuten, bevorzugt zwischen 6 und 45 Minuten und besonders bevorzugt zwischen 10 und 30 Minuten.

Nach der Durchführung des erfindungsgemäßen Verfahrens, gegebenenfalls nach Abkühlung trennt sich das Reaktionsgemisch in zwei Phasen auf. Die organische Phase enthält neben dem Lösungsmittel das hergestellte Dialkyldicarbonat und gegebenenfalls geringe Mengen nicht umgesetzten Halogenameisensäureester sowie den Katalysator. Die wässrige Phase enthält neben Wasser die gebildeten anorganischen Salze.

Das Produkt liegt bereits in hoher Reinheit vor, so dass neben der Phasentrennung keine destillative Trennung mehr notwendig ist.

Durch Verwendung des erfindungsgemäß eingesetzten Katalysators lassen sich Dialkyldicarbonate in hoher Ausbeute herstellen.

### Beispiele

### Beispiel 1

In einem Reaktionsgefäß wird ein Gemisch aus 18,87 g (0,2 mol) Chlorameisensäuremethylester (CAME) und 14,83 g Toluol vorgelegt und dann 3,57 g Barlox 12 (Lonza, Basel) (0,0045 mol) ((30 gew.%ige Lösung aus 70% Lauryldimethyldiaminoxid (Aminoxid der Formel (I) mit R¹ und R² = Methyl, R³ = C₁₂-Alkyl) 26% Myristaminoxid (Aminoxid der Formel (I) mit R¹ und R² = Methyl, R³ = C₁₄-Alkyl und 4% Hexadecanyldimethylaminoxid (Aminoxid der Formel (I) mit R¹ und R² = Methyl, R³ = C₁₆-Alkyl) in Wasser) und 62,73 g einer ca. 14%iger NaOH (0,22 mol) eingeleitet.

Dieses wird unter intensivem Rühren bei 17°C zur Reaktion gebracht. Nach ca. 20 min wurden die Phasen getrennt.

Die Rohausbeute liegt bei ca. 95% bezogen auf die eingesetzte Menge CAME.

### Vergleichsbeispiel 1 (mit einem tertiären Amin als Katalysator)

Das vorliegenden Beispiel ist in ähnlicher Form bereits aus der EP 1747185 A (Beispiel 3) bekannt.

In einem Reaktionsgefäß wird ein Gemisch aus 20,41 g (0,22 mol) CAME und 14,78 g Toluol vorgelegt und dann 0,54 g (0,0015 mol) Alamin 308 (Tris-isooctylamin) und 64,27 g einer ca. 14%iger NaOH (0,22 mol) eingeleitet.

Dieses wird unter intensivem Rühren bei 12°C zur Reaktion gebracht. Nach ca. 20 min wurden die Phasen getrennt.

Die Rohausbeute liegt bei ca. 91% bezogen auf die eingesetzte Menge CAME.

## Patentansprüche

1. Verfahren zur Herstellung von Dialkyldicarbonaten durch Umsetzung der entsprechenden Halogenameisensäurealkylester mit mindestens einem Alkalihydroxid, Erdalkalihydroxid und/oder -carbonat in Gegenwart von mindestens einem, mit Wasser nicht mischbaren organischen Lösungsmittel und in Gegenwart eines Katalysators **dadurch gekennzeichnet, dass** als Katalysator mindestens eine Verbindung der Formel (I) 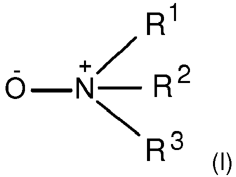 worin
R¹ und R² = geradkettiges oder verzweigtes C₁-C₆-Alkyl,
R³ = geradkettiges oder verzweigtes C₁₀-C₂₂-Alkyl,
wobei R¹ und R² jeweils unabhängig voneinander sind und gleich oder verschieden sein können, eingesetzt wird.

2. Verfahren gemäß Anspruch 1, **dadurch gekennzeichnet dass** als Katalysator mindestens eine Verbindung der Formel (I) gemäß Anspruch 1 eingesetzt wird, worin R³ jeweils geradkettiges oder verzweigtes Dodecanyl, Undecanyl, Tridecanyl, Tetradecanyl, Pentadecanyl, Hexadecanyl, Heptadecanyl, Octadecanyl, Nonadecanyl, Eicosanyl, Icosanyl, Heneicosanyl oder Dodoconyl ist.

3. Verfahren gemäß Anspruch 1 oder 2, **dadurch gekennzeichnet dass** als Katalysator eine Mischung der Verbindungen der Formel (I) eingesetzt wird, **dadurch gekennzeichnet, dass** die Katalysatoren verschiedene Reste R³ mit geradkettigem oder verzweigtem C₁₀-, C₁₁-, C₁₂-, C₁₃-, C₁₄-, C₁₅-, C₁₆-, C₁₇-, C₁₈-Alkyl aufweisen.

4. Verfahren gemäß wenigstens einem der Ansprüche 1 bis 3, **dadurch gekennzeichnet dass** als Katalysator mindestens eine Verbindung der Formel (I) gemäß Anspruch 1 eingesetzt wird, worin R¹ und R² = Methyl, Ethyl, n-Propyl, s-Propyl, n-Butyl, s-Butyl, i-Butyl oder t-Butyl ist und R³ = geradkettiges oder verzweigtes C₁₀-C₁₈ - Alkyl.

5. Verfahren gemäß wenigstens einem der Ansprüche 1 bis 4, **dadurch gekennzeichnet dass** als Katalysator mindestens eine Verbindung der Formel (I) gemäß Anspruch 1 eingesetzt wird, worin R¹ und R² = Methyl und R³ = C₁₀-C₁₆-Alkyl.

6. Verfahren gemäß wenigstens einem der Ansprüche 1 bis 5, **dadurch gekennzeichnet dass** als Katalysator mindestens eine Verbindung der Formel (I) gemäß Anspruch 1 eingesetzt wird, worin R¹ und R² = Methyl und R³ = Dodecanyl, Tetradecanyl, Hexadecanyl.

7. Verfahren gemäß wenigstens einem der Ansprüche 1 bis 6, **dadurch gekennzeichnet dass** als Verbindung der Formel (I) Mischungen mit verschiedenen Resten R³ mit geradkettigem oder verzweigtem C₁₂-, C₁₄- und C₁₆-Alkyl und R¹ und R² = Methyl eingesetzt werden und bei denen der Gehalt der Verbindung mit R³ = C₁₂-Alkyl von 60 Gew.% und 80 Gew.% und der Gehalt der Verbindung mit R³ = C₁₄-Alkyl von 19 Gew. % und 30 Gew. % und der Gehalt der Verbindung mit R³ = C₁₆-Alkyl von 1 Gew. % und 10 Gew.% bezogen auf das Gesamtgewicht der eingesetzten Verbindung der Formel (I) ist.

8. Verfahren gemäß wenigstens einem der Ansprüche 1 bis 7, **dadurch gekennzeichnet, dass** Dialkyldicarbonate der Formel (II) 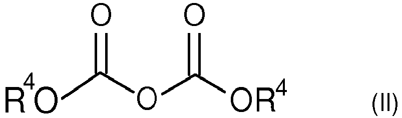 worin
R⁴ für geradkettiges oder verzweigtes C₁-C₂₀-Alkyl steht,
durch Umsetzung von Halogenameisensäurealkylester der Formel (III) 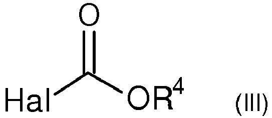 worin
Hal für Halogen, bevorzugt für F, Cl, Br, I, insbesondere für Chlor steht, und
R⁴ für geradkettiges oder verzweigtes C₁-C₂₀-Alkyl steht.

9. Verfahren gemäß Anspruch 8, **dadurch gekennzeichnet, dass** R⁴ für Methyl, Ethyl, n-Propyl, iso-Propyl, n-Butyl oder i-Butyl steht.

10. Verfahren gemäß wenigstens einem der Ansprüche 1 bis 9, **dadurch gekennzeichnet, dass** die Alkalihydroxide, Erdalkalihydroxide und/oder-carbonate in Form wässriger Lösungen einsetzt werden.

11. Verfahren gemäß wenigstens einem der Ansprüchen 1 bis 10, **dadurch gekennzeichnet, dass** man mindestens ein mit Wasser nicht mischbares organisches Lösungsmittel aus der Reihe der aliphatischen und aromatischen Kohlenwasserstoffe, der chlorierten Kohlenwasserstoffe, der Dialkylcarbonate oder der mit Wasser nicht mischbaren Ether und Ester einsetzt.

12. Verfahren gemäß wenigstens einem der Ansprüche 1 bis 11, **dadurch gekennzeichnet, dass** die Menge an eingesetzten Verbindungen der Formel (I) 0,1 bis 7 Mol-%, bezogen auf Halogenameisensäureester, beträgt.

13. Verfahren gemäß wenigstens einem der Ansprüche 1 bis 12, **dadurch gekennzeichnet, dass** die Reaktion bei einer Temperatur von 15°C und 19 °C (bei Normaldruck) des eingesetzten Halogenameisensäureesters durchgeführt wird.

14. Verfahren gemäß wenigstens einem der Ansprüche 1 bis 13, **dadurch gekennzeichnet, dass** die Umsetzung in kontinuierlicher Arbeitsweise durchgeführt wird.

15. Verfahren gemäß wenigstens einem der Ansprüche 1 bis 14, **dadurch gekennzeichnet, dass** nach Beendigung der Umsetzung die Aufarbeitung des Reaktionsgemisches zur Abtrennung des Dialkyldicarbonats durch Phasentrennung und anschließender mehrstufiger Destillation der organischen Phase erfolgt.

## Claims

1. Method for preparing dialkyl dicarbonates by reacting the corresponding alkyl haloformates with at least one alkali metal hydroxide, alkaline earth metal hydroxide and/or carbonate in the presence of at least one organic solvent immiscible with water and in the presence of a catalyst, **characterized in that** the catalyst used is at least one compound of the formula (I) 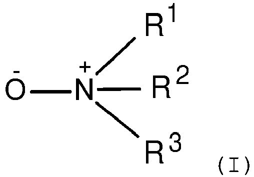 in which
R¹ and R² = straight-chain or branched C₁-C₆-alkyl,
R³ = straight-chain or branched C₁₀-C₂₂-alkyl,
wherein R¹ and R² are each mutually independent and may be the same or different.

2. Method according to Claim 1, **characterized in that** the catalyst used is at least one compound of the formula (I) according to Claim 1, in which R³ is in each case straight-chain or branched dodecanyl, undecanyl, tridecanyl, tetradecanyl, pentadecanyl, hexadecanyl, heptadecanyl, octadecanyl, nonadecanyl, eicosanyl, icosanyl, heneicosanyl or dodoconyl.

3. Method according to Claim 1 or 2, **characterized in that** the catalyst used is a mixture of the compounds of the formula (I), **characterized in that** the catalysts comprise different radicals R³ having straight-chain or branched C₁₀-, C₁₁-, C₁₂-, C₁₃-, C₁₄-, C₁₅-, C₁₆-, C₁₇-, C₁₈-alkyl.

4. Method according to at least one of Claims 1 to 3, **characterized in that** the catalyst used is at least one compound of the formula (I) according to Claim 1, in which R¹ and R² = methyl, ethyl, n-propyl, s-propyl, n-butyl, s-butyl, isobutyl or t-butyl and R³ = straight-chain or branched C₁₀-C₁₈- alkyl.

5. Method according to at least one of Claims 1 to 4, **characterized in that** the catalyst used is at least one compound of the formula (I) according to Claim 1, in which R¹ and R² = methyl and R³ = C₁₀-C₁₆- alkyl.

6. Method according to at least one of Claims 1 to 5, **characterized in that** the catalyst used is at least one compound of the formula (I) according to Claim 1, in which R¹ and R² = methyl and R³ = dodecanyl, tetradecanyl, hexadecanyl.

7. Method according to at least one of Claims 1 to 6, **characterized in that** the compound of the formula (I) used comprises mixtures having different radicals R³ having straight-chain or branched C₁₂-, C₁₄- and C₁₆-alkyl and R¹ and R² = methyl and in which the content of the compound where R³ = C₁₂-alkyl is from 60 % by weight and 80 % by weight and the content of the compound where R³ = C₁₄-alkyl is from 19 % by weight and 30 % by weight and the content of the compound where R³ = C₁₆-alkyl is from 1 % by weight and 10% by weight, based on the total weight of the compound of the formula (I) used.

8. Method according to at least one of Claims 1 to 7, **characterized in that** dialkyl dicarbonates of the formula (II) 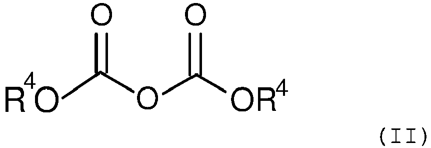 in which
R⁴ is straight-chain or branched C₁-C₂₀-alkyl, by reacting alkyl haloformate of the formula (III) 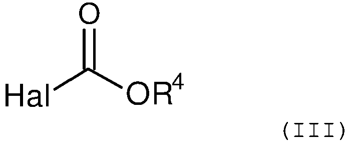 in which
Hal is halogen, preferably F, Cl, Br, I, especially chlorine, and
R⁴ is straight-chain or branched C₁-C₂₀-alkyl.

9. Method according to Claim 8, **characterized in that** R⁴ is methyl, ethyl, n-propyl, isopropyl, n-butyl or isobutyl.

10. Method according to at least one of Claims 1 to 9, **characterized in that** the alkali metal hydroxides, alkaline earth metal hydroxides and/or carbonates are used in the form of aqueous solutions.

11. Method according to at least one of Claims 1 to 10, **characterized in that** at least one organic solvent immiscible with water is used from the series of aliphatic and aromatic hydrocarbons, chlorinated hydrocarbons, dialkyl carbonates or ethers and esters immiscible with water.

12. Method according to at least one of Claims 1 to 11, **characterized in that** the amount of compounds of the formula (I) used is 0.1 to 7 mol%, based on haloformate.

13. Method according to at least one of Claims 1 to 12, **characterized in that** the reaction of the haloformate used is carried out at a temperature of 15°C and 19°C (at standard pressure).

14. Method according to at least one of Claims 1 to 13, **characterized in that** the reaction is carried out in a continuous mode of operation.

15. Method according to at least one of Claims 1 to 14, **characterized in that**, after completion of the reaction, the work-up of the reaction mixture for separating the dialkyl dicarbonate is effected by phase separation and subsequent multi-stage distillation of the organic phase.

## Revendications

1. Procédé pour la préparation de dicarbonates de dialkyle par transformation des esters d'alkyle d'acide halogénoformique correspondants avec au moins un hydroxyde de métal alcalin, un hydroxyde et/ou carbonate de métal alcalino-terreux en présence d'au moins un solvant organique non miscible à l'eau et en présence d'un catalyseur, **caractérisé en ce qu'**au moins un composé de formule (I) 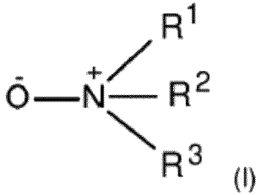 est utilisé en tant que catalyseur
dans laquelle
R¹ et R² = C₁₋₆₋alkyle linéaire ou ramifié,
R³ = C₁₀₋₂₂₋alkyle linéaire ou ramifié,
R¹ et R² étant à chaque fois indépendants l'un de l'autre et pouvant être identiques ou différents.

2. Procédé selon la revendication 1, **caractérisé en ce qu'**au moins un composé de formule (I) selon la revendication 1 est utilisé en tant que catalyseur, dans laquelle R³ est à chaque fois dodécanyle, undécanyle, tridécanyle, tétradécanyle, pentadécanyle, hexadécanyle, heptadécanyle, octadécanyle, nonadécanyle, éicosanyle, icosanyle, hénéicosanyle ou dodoconyle, linéaire ou ramifié.

3. Procédé selon la revendication 1 ou 2, **caractérisé en ce qu'**un mélange de composés de formule (I) est utilisé en tant que catalyseur, **caractérisé en ce que** les catalyseurs présentent différents radicaux R³ comportant un C₁₀-alkyle, C₁₁-alkyle, C₁₂-alkyle, C₁₃-alkyle, C₁₄-alkyle, C₁₅-alkyle, C₁₆-alkyle, C₁₇-alkyle, C₁₈-alkyle, linéaire ou ramifié.

4. Procédé selon au moins l'une des revendications 1 à 3, **caractérisé en ce qu'**au moins un composé de formule (I) selon la revendication 1 est utilisé en tant que catalyseur, dans laquelle R¹ et R² = méthyle, éthyle, n-propyle, s-propyle, n-butyle, s-butyle, i-butyle ou t-butyle et R³ = C₁₀₋₁₈-alkyle linéaire ou ramifié.

5. Procédé selon au moins l'une des revendications 1 à 4, **caractérisé en ce qu'**au moins un composé de formule (I) selon la revendication 1 est utilisé en tant que catalyseur, dans laquelle R¹ et R² = méthyle et R³ = C₁₀₋₁₆-alkyle.

6. Procédé selon au moins l'une des revendications 1 à 5, **caractérisé en ce qu'**au moins un composé de formule (I) selon la revendication 1 est utilisé en tant que catalyseur, dans laquelle R¹ et R² = méthyle et R³ = dodécanyle, tétradécanyle, hexadécanyle.

7. Procédé selon au moins l'une des revendications 1 à 6, **caractérisé en ce qu'**en tant que composé de formule (I), des mélanges comportant différents radicaux R³ comportant un C₁₂-alkyle, C₁₄-alkyle, et C₁₆-alkyle, linéaire ou ramifié et R¹ et R² = méthyle sont utilisés et pour lesquels la teneur du composé comportant R³ = C₁₂-alkyle est de 60 % en poids et 80 % en poids et la teneur du composé comportant R³ = C₁₄-alkyle est de 19 % en poids et 30 % en poids et la teneur du composé comportant R³ = C₁₆-alkyle est de 1 % en poids et 10 % en poids par rapport au poids total du composé utilisé de formule (I).

8. Procédé selon au moins l'une des revendications 1 à 7, **caractérisé en ce que** des dicarbonates de dialkyle de formule (II) 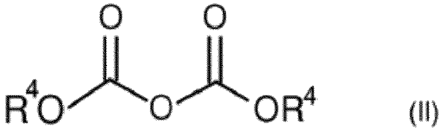 dans laquelle
R⁴ représente C₁₋₂₀-alkyle linéaire ou ramifié,
par transformation d'esters d'alkyle d'acide halogénoformique de formule (III) 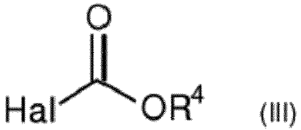 dans laquelle
Hal représente un halogène, préférablement F, Cl, Br, I, en particulier chlore, et
R⁴ représente C₁₋₂₀-alkyle linéaire ou ramifié.

9. Procédé selon la revendication 8, **caractérisé en ce que** R⁴ représente méthyle, éthyle, n-propyle, iso-propyle, n-butyle ou i-butyle.

10. Procédé selon au moins l'une des revendications 1 à 9, **caractérisé en ce que** les hydroxydes de métal alcalin, les hydroxydes et/ou carbonates de métal alcalino-terreux sont utilisés sous forme de solutions aqueuses.

11. Procédé selon au moins l'une des revendications 1 à 10, **caractérisé en ce qu'**on utilise au moins un solvant organique non miscible à l'eau de la série des hydrocarbures aliphatiques et aromatiques, des hydrocarbures chlorés, des carbonates de dialkyle ou des éthers et esters non miscibles à l'eau.

12. Procédé selon au moins l'une des revendications 1 à 11, **caractérisé en ce que** la quantité de composés utilisés de formule (I) est de 0,1 à 7 % en moles, par rapport à l'ester d'acide halogénoformique.

13. Procédé selon au moins l'une des revendications 1 à 12, **caractérisé en ce que** la réaction est mise en œuvre à une température de 15 °C et 19 °C (à pression normale) de l'ester d'acide halogénoformique utilisé.

14. Procédé selon au moins l'une des revendications 1 à 13, **caractérisé en ce que** la transformation est mise en œuvre dans une mode de fonctionnement continu.

15. Procédé selon au moins l'une des revendications 1 à 14, **caractérisé en ce qu'**après la fin de la transformation, le traitement du mélange de réaction est réalisé pour la séparation du dicarbonate de dialkyle par séparation de phase et ultérieurement par distillation en plusieurs étapes de la phase organique.
